**Europäisches Patentamt**

**European Patent Office**

**Office européen des brevets**

(19)

(11) Publication number: **0 013 444**
**B1**

(12)

# EUROPEAN PATENT SPECIFICATION

(45) Date of publication of patent specification: **04.08.82**

(51) Int. Cl.³: **C 07 D 323/00**

(21) Application number: **79200636.3**

(22) Date of filing: **01.11.79**

(54) Complexes between 1,4,7,10,13,16-hexaoxacyclooctadecane (18-crown-6) and dimethylcarbonate or dimethyloxalate and process for isolating the macrocyclic polyether via said complexes.

(30) Priority: **10.11.78 GB 4394578**

(43) Date of publication of application:
**23.07.80 Bulletin 80/15**

(45) Publication of the grant of the patent:
**04.08.82 Bulletin 82/31**

(84) Designated Contracting States:
**BE CH DE FR GB IT NL SE**

(56) References cited:
**FR - A - 2 116 050**
**US - A - 3 562 295**
**US - A - 3 997 563**

(73) Proprietor: **SHELL INTERNATIONALE RESEARCH MAATSCHAPPIJ B.V.**
**Carel van Bylandtlaan 30**
**NL-2596 HR Den Haag (NL)**

(72) Inventor: **Van Zon, Arie**
**Badhuisweg 3**
**NL-1031 CM Amsterdam (NL)**

(74) Representative: **Puister, Antonius Tonnis, Mr. et al,**
**P.O. Box 302**
**NL-2501 CH The Hague (NL)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

**0 013 444**

Complexes between 1,4,7,10,13,16-hexaoxacyclooctadecane (18-crown-6) and dimethylcarbonate or dimethyloxalate and process for isolating the macrocyclic polyether via said complexes

The present invention relates to novel complexes of the macrocyclic polyether 1, 4, 7, 10, 13, 16-hexaoxacyclooctadecane (hereinafter also referred to as 18-crown-6), and to a method for the isolation of 18-crown-6 from mixtures comprising this macrocyclic polyether in which the novel complexes are advantageously employed to effect separation of the macrocyclic polyether from the mixture.

Since 18-crown-6 has special utility in the complexing of lower alkali metal cations, e.g. potassium, a considerable technical interest in 18-crown-6 has arisen and, as a result, a number of synthetic techniques has been proposed for preparing this macrocyclic polyether. Among those, reference may be made to the catalytic oligomerization of ethylene oxide as described in U.S. Patent 3,928,388 and the reaction of tetraethylene glycol with bis(2-chloroethyl)ether in the presence of potassium hydroxide and tetrahydrofuran without addition of water as described in "Synthesis" 1976, 515—516.

The technique disclosed for isolating 18-crown-6 from the reaction mixture obtained by the oligomerization process referred to hereinabove involves chromatographic separation on acid-washed alumina or silica and elution with readily volatile hydrocarbons. This isolation procedure is not particularly attractive from a commercial point of view because the absorptions capacities of alumina or silica for 18-crown-6 are rather low and the used alumina or silica must be regenerated or discarded and replaced by fresh alumina or silica.

The main disadvantage of the preparation as described in "Synthesis 1976, 515—516 is that 18-crown-6 has to be distilled overhead, and therefore additional measures have to be taken to avoid the occurrence of powerful and destructive explosions which are known to occur during the distillation of 18-crown-6 (see "Chemical and Engineering News", September 6, 1976, page 5 and December 13, 1976, page 5). Moreover, the isolation of 18-crown-6 via its complex with acetonitrile has the disadvantage that very low temperatures (−45°C) must be used to precipitate the 18-crown-6-acetonitrile complex from the excess acetonitrile because of the high solubility of the 18-crown-6-acetonitrile complex in acetonitrile.

The isolation of 18-crown-6 from mixtures comprising this compound can also be carried out using nitromethane as the complexing agent as described and claimed in published European Patent Application No. 0 000 218. The isolation can be carried out at ambient or somewhat lower temperature (because of the lower solubility of the 18-crown-6-nitromethane complex in nitromethane) and 18-crown-6 need not to be distilled in order to separate it from the 18-crown-6-nitromethane complex as nitromethane can be removed from the complex by heating the complex at sub-atmospheric pressure.

However, it should be noted that the use of nitromethane implies some drawbacks, especially when intended in large scale operations due to its toxicity and explosion danger when subjected to higher temperatures.

It has now been found that 18-crown-6 will form complexes with dimethylcarbonate or dimethyloxalate which can be advantageously employed to isolate 18-crown-6 from mixtures comprising this compound, including mixtures obtained as reaction products in the preparation of 18-crown-6, for instance in the processes referred to hereinabove.

The present invention therefore relates to novel complexes formed between 1, 4, 7, 10, 13, 16-hexaoxacyclooctadecane (18-crown-6) and dimethylcarbonate or dimethyloxalate containing one molecule of dimethylcarbonate or dimethyloxalate per molecule of 18-crown-6 as well as to a process for isolating 18-crown-6 from mixtures comprising 18-crown-6 by treating 18-crown-6 with dimethylcarbonate or dimethyloxalate to precipitate a complex of 18-crown-6 and dimethylcarbonate or dimethyloxalate, separating the precipitated complex from the resulting solution of non-complexed material and affording uncomplexed 18-crown-6 by dissociating the separated complex.

The process according to the present invention is advantageous in that 18-crown-6 can not only be isolated with high efficiency but also in high purity (> 98% and often more than 99.5% (calculated on starting amount of 18-crown-6)) as dimethylcarbonate and dimethyloxalate selectively form complexes with 18-crown-6, even in the presence of one or more other macrocyclic polyethers. Moreover, 18-crown-6 can be easily obtained from the separated complexes since dimethylcarbonate and dimethyloxalate can be removed quantitatively by keeping the complexes under reduced pressure, e.g. at a pressure between 5 Pa and 6 kPa at room temperature or at moderate temperature.

The novel 18-crown-6-dimethylcarbonate complex according to the present invention can be suitably prepared by treating 18-crown-6 either as such or when present in a mixture with dimethylcarbonate at a temperature between −30°C and +30°C, preferably between 0°C and +20°C.. The novel 18-crown-6-dimethyloxalate complex according to the present invention can be suitably prepared by treating a mixture comprising 18-crown-6 with dimethyloxalate at temperatures between +40°C and −30°C, preferably between +30°C and 0°C. With dimethylcarbonate best results are obtained at temperatures below +10°C, e.g. at +4°C whereas excellent results with dimethyloxalate are already obtained at ambient temperature.

The 18-crown-6-complex can be easily separated from the solution containing non-complexed

2

material by any conventional liquid-solid separation technique, for example by filtration, centrifugation or decantation.

As the novel complexes appear to be of the 1:1 type, i.e. one mole of 18-crown-6 per mole of dimethylcarbonate or dimethyloxalate, 18-crown-6 is normally reacted with the complexing agents using a molar ratio of complexing agent to 18-crown-6 of not less than 1. Preferably, the molar ratio applied is not higher than 10:1 although this range is not critical. An additional advantage inherent with the use of dimethylcarbonate or dimethyloxalate is that only an equimolar amount of the complexing agent has to be removed from the separated complex compared with two moles of nitromethane to be removed per mole of 18-crown-6-nitromethane complex.

The process according to the present invention is of particular interest in that it affords the selective isolation of 18-crown-6 from mixtures containing this compound, even in the presence of one or more other macrocyclic polyethers having more than 7 carbon atoms in the ring such as 1, 4, 7, 10, 13-pentaoxacyclopentadecane (15-crown-5).

The process according to the present invention is preferably carried out in an environment which contains a solvent wherein 18-crown-6 is soluble and wherein the solubility of the resulting complex is substantially lower. For purposes of this description, a solvent is taken to consist substantially of a specified compound when the content of this specific compound in the solvent is more than 50%w.

A particular good class of solvents comprises the class of dialkyl ethers for instance, di-ethylether, di-n-butylether and methyl-t-butylether. Solid complexes are normally formed immediately when using the above-mentioned ethers. Also dialkyl diethers such as dimethoxyethane and diethoxy ethane can be used advantageously as a solvent.

Also cyclic ethers (other than a macrocyclic polyether having more than 7 carbon atoms in the ring) such as the dioxanes and tetrahydrofuran can be used as solvents, preferably in relative small amounts and at lower temperatures. Dimethylcarbonate itself can also be used as a solvent in the process for the preparation of the 18-crown-6-dimethylcarbonate complex.

The 18-crown-6 complexes may also be formed when alkanols, such as methanol, ethanol, i-propanol or 1,2-dihydroxyethane are used as solvents. Further solvents comprise ketones such as acetone and methylethylketone as well as hydrocarbons or mixtures of hydrocarbons (e.g. benzene, toluene or the xylenes and aliphatic hydrocarbons such as hexane and heptane. Mixtures of solvents may also be used. When applying alkanols, ketones or hydrocarbons the complexes are best formed by keeping the mixtures at least a couple of hours at lower temperatures. No complexes were obtained when using water as the solvent. However, amounts of water up to 25% can be tolerated when using water-miscible solvents.

The 18-crown-6 in the starting mixture may be treated with the complexing agent in any suitable manner, for example by adding the complexing agent to the starting mixture and, if desired, cooling the mixture thus obtained to a temperature at which the complex readily precipitates, or by dissolving the starting mixture in a suitable solvent and adding the complexing agent whether or not in the presence of a solvent at ambient or somewhat lower temperature to the solution obtained. Alternatively, the starting mixture may be treated with a solution of the complexing agent in a suitable solvent. The starting mixture may be solid or liquid at the temperature at which 18-crown-6 is treated with dimethylcarbonate.

As discussed hereinbefore, dimethylcarbonate and dimethyloxalate can isolate 18-crown-6 from mixtures comprising 18-crown-6 whether or not in the presence of one or more other related macrocyclic polyethers. High efficiencies in the recovery of pure 18-crown-6 have been achieved starting from unpurified reaction mixtures containing about 15%w (e.g. as obtained by the catalytic oligomerization of ethylene oxide) of 18-crown-6 as well as from mixtures containing about 50%w of 18-crown-6, even when the remaining 50%w of the mixture consists of the closely related compound 15-crown-5. It is also possible to isolate 18-crown-6 from reaction mixtures wherein an alkalimetal halide, e.g. potassium chloride is also present, either as such or complexed with 18-crown-6. The reaction mixture should then be treated after the complex formation with the complexing agents with a solvent wherein the complex is soluble e.g. with methylene chloride in order to remove by filtration the alkalimetal salt. It is also possible to add the complexing agent and the desired solvent together.

The 18-crown-6 complexes according to the present invention can be readily dissociated into 18-crown-6 and the complexing agent by treating them at sub-atmospheric pressure if desired under gentle heating, e.g. to temperatures up to 80°C and removing the complexing agent. Normally pressures between 5 and 1 kPa will be sufficient but lower pressure can also be applied. It is also possible to decompose the 18-crown-6 complexes, especially the 18-crown-6-dimethylcarbonate complex by heating at atmospheric pressure without distilling 18-crown-6.

The invention will now be illustrated by reference to the following Examples.

Example 1

a) *18-crown-6-dimethylcarbonate complex*

5 Equivalents of dimethylcarbonate were added to a solution of 53 mg 18-crown-6 in 1 ml diethylether at 25°C. A solid complex was formed immediately. The precipitated crystals were filtered

off at atmospheric pressure. The crystals contained 81% of the starting amount of 18-crown-6. A melting point range of 39—54°C was observed.

The NMR spectrum of the crystals, recorded at 90 MHz in deuterochloroform solution, showed—relative to a tetramethylsilanestandard—an absorption at $\delta = 3.688$ ppm, indicating the presence of 18-crown-6, and an absorption at $\delta = 3.792$ ppm, indicating the presence of dimethylcarbonate. The molar ratio of 18-crown-6 to dimethylcarbonate in the 18-crown-6-dimethylcarbonate complex, calculated from the NMR spectrum was 1:1.01 ($\pm$ 0.05). Dimethylcarbonate was removed quantitatively from the complex by keeping it under reduced pressure (2.6 kPa) for half an hour at room temperature. A similar experiment was carried out by forming the complex at +4°C. Again a solid complex was formed immediately. The crystals obtained after filtration contained 85% of the starting amount of 18-crown-6.

b) *18-crown-6-dimethyloxalate complex*

2 Equivalents of dimethyloxalate were added to a solution of 53 mg 18-crown-6 in 1 ml of diethylether at 25°C. A solid complex was formed immediately. The precipitated crystals were filtered off at a atmospheric pressure and washed with 1 ml of a 0.425 M solution of dimethyloxalate in diethylether. The crystals contained 80% of the starting amount of 18-crown-6. A melting point range of 62—82°C was observed.

The NMR spectrum of the crystals, recorded at 90 MHz in deuterochloroform solution, showed—relative to a tetramethylsilanestandard—an absorption at $\delta = 3.688$, indicating the presence of 18-crown-6, and an absorption at $\delta = 3.919$, indicating the presence of dimethyloxalate. The molar ratio of 18-crown-6 to dimethyloxalate in the 18-crown-6-dimethyloxalate complex, calculated from the NMR spectrum was 1:0.96.

Dimethyloxalate was removed quantitatively from the complex by keeping it at 50 Pa for two hours at 70°C.

Example 2

*Solid complex formation in various solvents*

Solid complexes were formed when dimethylcarbonate (5 eq.[x]) or dimethyloxalate (2 eq.) was added to a solution of 18-crown-6 (0.2 M) in the solvents tabulated in Table I at the temperatures indicated therein. The recovery (%) of 18-crown-6 after 24 hours as well as the molar ratios of 18-crown-6 to dimethylcarbonate, respectively dimethyloxalate in the respective complexes (determined by NMR spectroscopy) are also given in Table I. The complexing agents were removed from the complexes in the manner as indicated in Example 1.

x) By 5 eq. is meant 5 times the molar amount of 18-crown-6 present in the solution.

TABLE I

| Solvent | Recovery of 18-crown-6 (%) from dimethylcarbonate complex after 24 hours at | | | Recovery of 18-crown-6 (%) from dimethyloxalate complex after 24 hours at | | | |
|---|---|---|---|---|---|---|---|
| | 25°C | 4°C | Molar ratio | 25°C | 5°C | −20°C | Molar ratio |
| $(nC_4H_9)_2O$ | 85 | 95 | 1:1.10 | 71[o] | | | 1:0.97 |
| $CH_3O\ t\text{-}C_4H_9$ | 57[x] | 87 | 1:1.01 | 73 | | | 1:0.99 |
| $(CH_3O)_2CO$ | — | 73[x] | 1:0.96 | | | | |
| $CH_3OCH_2CH_2OCH_3$ | — | 49[x] | 1:0.94 | — | 52 | | 1:0.99 |
| $CH_3OH$ | — | 18[x] | 1:0.98 | — | 49 | | 1:1.00 |
| THF | 50[+] | | 1:1.06 | — | — | 59 | 1.1.14 |

x) solid complex formed over a period of 1—4 hours
+) solid complex formed when applying higher concentrations
—) no solid complex formed within 24 hours
o) experiment carried out at higher dilution (2x) because of limited solubility of complexing agent.

Solid complexes were also obtained using n-heptane (dimethylcarbonate, 25°C, rec. 52%, molar ratio 1:0.96 and dimethyloxalate, 25°C, rec. 50%, molar ratio 1:1.04). When applying higher concentrations (5x) complexes were obtained using benzene (dimethylcarbonate, 25°C, rec. 59%, molar ratio 1:1.07) and 1,4-dioxane (dimethylcarbonate, 25°C, rec. 37% molar ratio 1:0.98 and dimethyloxalate, 25°C, rec 50%, molar ratio 1:0.96).

Example 3

*Isolation of 18-crown-6 from mixtures using dimethylcarbonate*

18-crown-6 was isolated from mixtures comprising this compound via the 18-crown-6-dimethylcarbonate complex by selective complexation with dimethylcarbonate at 4°C. The 18-crown-6 containing mixtures were:

A: a crude reaction mixture containing 15% (w/w) 18-crown-6;

B: a reaction mixture containing 50% (w/w) 18-crown-6;

and

C: a mixture (1:1) of 15-crown-5 and 18-crown-6.

The purity of the 18-crown-6 obtained after decomposition of the respective complexes was in all cases ≥98%. The solvents employed, the amounts of reaction mixture and dimethylcarbonate used as well as the efficiency of the isolation procedure i.e. the percentage of 18-crown-6 recovered after decomposition of the complexes as described in Example 1 (a) are given in Table II.

TABLE II

| Mixture containing 18-crown-6 (g) | Solvent (ml) | Dimethylcarbonate (ml) | Efficiency (%) |
|---|---|---|---|
| A—(3.4) | $(C_2H_5)_2O$—(5.4) | 1.0 | 82 |
| A—(3.0) | — | 8.0 | 85 |
| B—(1.0) | $(C_2H_5)_2O$—(2.0) | 0.5 | 80 |
| B—(1.0) | $CH_2O$—$tC_4H_9$—(2.0) | 0.5 | 84 |
| B—(1.0) | — | 2.0 | 80 |
| C—(0.1) | $(C_2H_5)_2O$—(1.0) | 0.1 | 85 |

Example 4

*Isolation of 18-crown-6 from mixtures using dimethyloxalate*

A reaction mixture (1 g) containing 50% 18-crown-6 was treated with a solution of dimethyloxalate (0.33 g) in diethylether (3 ml) at room temperature. The precipitated complex was separated by filtration and washed with a solution of dimethyloxalate (0.2 g) in diethylether (2 ml). The purity of the crystals of the 18-crown-6-dimethyloxalate complex obtained in 81% yield, calculated on the starting amount of 18-crown-6, was >98%.

This procedure was repeated using the mixtures A and C as described in Example 3. The experimental details and the efficiency achieved are tabulated in Table III.

TABLE III

| Mixture containing 18-crown-6 (g) | $(C_2H_5)_2O$ solvent (ml) | Dimethyloxalate (g) | Efficiency (%) |
|---|---|---|---|
| A—(2.2) | 5 | 0.5 | 80 |
| C—(0.2) | 2 | 0.2 | 86 |
| D[x]—(5.0) | 10 | 2.2 | 92 |

x unpurified reaction mixture from 18-crown-6 synthesis according to J. C. S. Chem. Commun. (1978)504 containing 16% 18-crown-6

**0 013 444**

## Claims

1. The complexes between 1, 4, 7, 10, 13, 16-hexaoxacyclooctadecane (18-crown-6) and dimethylcarbonate and dimethyloxalate containing one molecule of dimethylcarbonate or dimethyloxalate per molecule of 18-crown-6.

2. Process for isolating 18-crown-6 from mixtures containing 18-crown-6 which comprises treating 18-crown-6 with dimethylcarbonate or dimethyloxalate, separating the precipitated complex from the resulting solution of non-complexed material and affording uncomplexed 18-crown-6 by dissociating the separated complex.

3. Process according to claim 2, in which the reaction is carried out in the presence of a solvent for 18-crown-6.

4. Process according to claim 3, in which the solvent substantially consists of an ether (other than a macrocyclic polyether having more than 7 carbon atoms in the ring), an alkanol or a ketone.

5. Process according to claim 4, in which a dialkylether, preferably diethylether, di(n-butyl)ether, or methyl-t-butylether is used as the solvent.

6. Process according to any one of claims 2—5, in which the complex formation between 18-crown-6 and dimethylcarbonate is carried out at a temperature in the range of from −30°C to +30°C, preferably from 0°C to +20°C.

7. Process according to any one of claims 2—5, in which the complex formation between 18-crown-6 and dimethyloxalate is carried out at a temperature between −30°C and +40°C, preferably from 0°C to +30°C.

8. Process according to any one of claims 2—7, in which 18-crown-6 is treated with dimethylcarbonate or dimethyloxalate using a molar ratio of dimethylcarbonate or dimethyloxalate to 18-crown-6 in the range of from 1:1 to 10:1.

9. Process according to any one of claims 2—8, in which the 18-crown-6 containing starting mixture contains one or more other macrocyclic polyethers having more than 7 carbon atoms in the ring.

10. Process according to any one of claims 2—9, in which the separated complex is dissociated at sub-atmospheric pressure at a temperature not exceeding +80°C.

## Revendications

1. Les complexes entre le 1, 4, 7, 10, 13, 16-hexaoxacyclooctadécane (18-couronne-6) et le carbonate de diméthyle ou l'oxalate de diméthyle contenant une molécule de carbonate de diméthyle ou d'oxalate de diméthyle par molécule de 18-couronne-6.

2. Procédé pour isoler le 18-couronne-6 à partir de mélanges contenant du 18-couronne-6, caractérisé en ce qu'on traite le 18-couronne-6 par le carbonate de diméthyle ou l'oxalate de diméthyle, on sépare le complexe précipité de la solution résultant de matière non complexée et on recueille du 18-couronne-6 non complexé en dissociant le complex séparé.

3. Procédé selon la revendication 2, caractérisé en ce que la réaction est conduite en présence d'un solvant pour le 18-couronne-6.

4. Procédé selon la revendication 3, caractérisé en ce que le solvant est constitué essentiellement d'un éther (autre qu'un polyéther macrocyclique ayant plus de 7 atomes de carbone dans le cycle), un alcanol ou une cétone.

5. Procédé selon la revendication 4, caractérisé en ce qu'on utilise comme solvant un oxyde d'alcoyle, de préférence l'oxyde d'éthyle, l'oxyde de n-butyle ou l'oxyde de méthyle et de t-butyle.

6. Procédé selon l'une quelconque des revendications 2 à 5, caractérisé en ce que la formation du complexe entre le 18-couronne-6 et le carbonate de diméthyle est effectuée à une température comprise entre −30°C et +30°C, de préférence entre 0°C et +20°C.

7. Procédé selon l'une quelconque des revendications 2 à 5, caractérisé en ce que la formation du complexe entre le 18-couronne-6 et l'oxalate de diméthyle est effectúee à une température comprise entre −30°C et +40°C, de préférence entre 0°C et +30°C.

8. Procédé selon l'une quelconque des revendications 2 à 7, caractérisé en ce qu'on traite le 18-couronne-6 par le carbonate de diméthyle ou l'oxalate de diméthyle en utilisant un rapport molaire du carbonate de diméthyle ou de l'oxalate de diméthyle au 18-couronne-6 comprise entre 1:1 et 10:1.

9. Procédé selon l'une quelconque des revendications 2 à 8, caractérisé en ce que le mélange de départ contenant du 18-couronne-6 contient un ou plusieurs autres polyéthers macrocycliques ayant plus de 7 atomes de carbone dans le cycle.

10. Procédé selon l'une quelconque des revendications 2 à 9, caractérisé en ce que le complexe séparé est dissocié à une pression inférieure à la pression atmosphérique à une température ne dépassant pas +80°C.

**Patentansprüche**

1. Die Komplexe zwischen 1, 4, 7, 10, 13, 16-Hexaoxacyclooctadecan (18-Krone-6) und Dimethylcarbonat oder Dimethyloxalat enthaltend 1 Molekül Dimethylcarbonat oder Dimethyloxalat je Molekül 18-Krone-6.

2. Verfahren zum Isolieren von 18-Krone-6 aus Gemischen enthaltend 18-Krone-6, dadurch gekennzeichnet, dass 18-Krone-6 mit Dimethylcarbonat oder Dimethyloxalat behandelt, der ausgefallene Komplex von der entstehenden Lösung von nicht komplex gebundenem Material abgetrennt und nicht komplex gebundenes 18-Krone-6 durch Dissoziieren des abgetrennten Komplexes isoliert wird.

3. Verfahren nach Anspruch 2, dadurch gekennzeichnet, dass die Reaktion in Gegenwart eines Lösungsmittels für 18-Krone-6 ausgeführt wird.

4. Verfahren nach Anspruch 3, dadurch gekennzeichnet, dass das Lösungsmittel im wesentlichen aus einem Äther (ein anderer Äther als ein macrocyclischer Polyäther mit mehr als 7 Kohlenstoffatomen im Ring), einem Alkanol oder einem Keton besteht.

5. Verfahren nach Anspruch 4, dadurch gekennzeichnet, dass ein Dialkyläther, vorzugsweise Diäthyläther, Di(n-butyl)äther oder Methyl-tert.-butyläther als Lösungsmittel verwendet wird.

6. Verfahren nach irgendeinem der Ansprüche 2 bis 5, dadurch gekennzeichnet, dass die Komplexbildung zwischen 18-Krone-6 und Dimethylcarbonat bei einer Temperatur im Bereich von —30°C bis +30°C, vorzugsweise von 0°C bis +20°C ausgeführt wird.

7. Verfahren nach irgendeinem der Ansprüche 2 bis 5, dadurch gekennzeichnet, dass die Komplexbildung zwischen 18-Krone-6 und Dimethyloxalat bei einer Temperatur zwischen —30°C und +40°C, vorzugsweise von 0°C bis +30°C ausgeführt wird.

8. Verfahren nach irgendeinem der Ansprüche 2 bis 7, dadurch gekennzeichnet, dass 18-Krone-6 mit Dimethylcarbonat oder Dimethyloxalat in einem Molverhältnis von Dimethylcarbonat oder Dimethyloxalat zu 18-Krone-6 im Bereich von 1:1 bis 10:1 behandelt wird.

9. Verfahren nach irgendeinem der Ansprüche 2 bis 8, dadurch gekennzeichnet, dass das 18-Krone-6 enthaltende Ausgangsgemisch ein oder mehrere andere macrocyclische Polyäther mit mehr als 7 Kohlenstoffatomen im Ring enthält.

10. Verfahren nach irgendeinem der Ansprüche 2 bis 9, dadurch gekennzeichnet, dass der abgetrennte Komplex bei einem Druck unter Atmosphärendruck bei einer Temperatur nicht über +80°C dissoziiert wird.